# EUROPEAN PATENT SPECIFICATION

(11) **EP 2 407 176 B1**
(45) Date of publication and mention of the grant of the patent: **19.10.2016**
(21) Application number: 10750713.9
(22) Date of filing: 02.03.2010
(51) Int. Cl.: A61K 9/00, A61K 9/08, A61K 9/72, A61K 38/44, A61K 47/24, A61K 47/26, A61K 47/48, A61P 11/00

(54) **AMELIORATING AGENT FOR CHRONIC OBSTRUCTIVE PULMONARY DISEASE**
LINDERUNGSMITTEL FÜR CHRONISCH-OBSTRUKTIVE LUNGENERKRANKUNG
AGENT D'AMÉLIORATION POUR LA BRONCHOPNEUMOPATHIE CHRONIQUE OBSTRUCTIVE

(30) Priority: 13.03.2009 JP 2009061075
(43) Date of publication of application: 18.01.2012
(73) Proprietor: LTT Bio-Pharma Co., Ltd., Minato-ku Tokyo 105-0022 (JP)
(72) Inventor: MIZUSHIMA, Toru, Kumamoto-shi Kumamoto 862-0975 (JP)
(74) Representative: Beckmann, Claus
(86) International application number: PCT/JP2010/053317
(87) International publication number: WO 2010/103959

(56) References cited:
- EP-A1- 1 188 445
- JP-A- 6 054 681
- JP-A- 2001 064 199
- KINNULA VUOKKO L ET AL: "Superoxide dismutases in the lung and human lung diseases.", AMERICAN JOURNAL OF RESPIRATORY AND CRITICAL CARE MEDICINE 15 JUN 2003 LNKD- PUBMED:12796054, vol. 167, no. 12, 15 June 2003 (2003-06-15), pages 1600-1619, XP002685116, ISSN: 1073-449X
- IGARASHI R ET AL: "LECITHINIZATION OF SUPEROXIDE DISMUTASE POTENTIATES ITS PROTECTIVE EFFECT AGAINST FORSSMAN ANTISERUM-INDUCED ELEVATION IN GUINEA-PIG AIRWAY RESISTANCE", JOURNAL OF PHARMACOLOGY AND EXPERIMENTAL THERAPEUTICS, THE AMERICAN SOCIETY FOR PHARMACOLOGY AND EXPERIMENTAL THERAPEUTICS, US, vol. 262, no. 3, 1 January 1992 (1992-01-01), pages 1214-1219, XP009108879, ISSN: 0022-3565
- BARNES, P.J. ET AL.: 'Prospects for new drugs for chronic obstructive pulmonary disease' LANCET vol. 364, no. 9438, 2004, pages 985 - 996, XP005066747
- MASARU SAGAI ET AL.: 'Diesel Haiki Biryushi (DEP) no Mouse eno Kikannai Toyo ni yoru Zensoku-yo Byotai no Hatsugen ni Tsuite Tokuni, Kassei Sanso Sansei o Kaishita Eikyo o Chushin to Shite' JOURNAL OF JAPAN SOCIETY OF AIR POLLUTION vol. 28, no. 4, 1993, pages 220 - 230, XP008167408

## Description

The present invention relates to an ameliorating agent for use in treating chronic obstructive pulmonary disease, and in particular, to an ameliorating agent for use in treating chronic obstructive pulmonary disease containing a lecithinized superoxide dismutase (hereinafter may be simply referred to as PC-SOD) as an active ingredient.

### BACKGROUND ART

A superoxide dismutase (hereinafter may be simply referred to as SOD) is a bioactive protein which was extracted from bovine blood as an anti-inflammatory protein by Huber et al., in 1965, and which has been found that it specifically eliminates superoxide anions (O₂⁻) as one of active oxygen species. In the living organism, active oxygen is mainly released from phagocytes such as neutrophils and macrophages for sterilization. In general, there are various antioxidants such as SODs for excessive active oxygen, which protect healthy cells against injury by the active oxygen.

However, when there is excessive active oxygen beyond the antioxidant ability of the antioxidants such as SODs, substances near the active oxygen, particularly cell membranes are attacked by the active oxygen to develop various disease conditions. In fact, since it has been proven that the active oxygen has potent tissue damage properties, it has been found that it becomes causative or precipitating factors of many disease conditions such as inflammation, allergy, tissue damage caused by ischemia reperfusion, and pulmonary fibrosis caused by an anticancer agent.

Under such a circumstance, a SOD which specifically eliminates active oxygen has been found, and the possibility of clinical application has been widely investigated. The inventors also have earnestly studied clinical usability of SOD. At this time, they have believed that, in order to enhance clinical effects of SOD, it is important to reduce clearance from the kidney and maintain a blood level of SOD, and to increase affinity for cell membranes and eliminate excessive active oxygen on the cell membranes. Thus, they have studied various modified SODs, and proposed a lecithinized superoxide dismutase (PC-SOD) (Patent Literatures 1 and 9).

The PC-SOD is a lecithinized SOD obtained by preparing a human Cu/Zn-superoxide dismutase (SOD) by gene recombination technology, and then chemically binding an average of four molecules of lecithin derivative (phosphatidylcholine derivative: PC) to one molecule of SOD (dimer). The PC-SOD has high affinity for cell membranes, and is approved to have high therapeutic effects on diseases involving active oxygen, such as ischemia-reperfusion injury and cardiomyopathy induced by anthracycline anticancer agents which are etiological factors in the lesions. Various agents containing PC-SOD as an active ingredient, such as a therapeutic agent for acute heart failure (Patent Literature 2), an antiviral agent (Patent Literature 3), a therapeutic agent for lupus nephritis (Patent Literature 4), an ameliorating agent for cerebral vascular accident-related dysfunction (Patent Literature 5), an anti-fibrosis agent (Patent Literature 6), or a treatment agent for allergic diseases (Patent Literature 7), a therapeutic agent for burns (Patent Literature 8), and a therapeutic agent for interstitial pneumonia (Patent Literature 10), have been already proposed.

Chronic obstructive pulmonary disease (COPD) is a disease, in which chronic inflammation in the lung is caused by various factors, particularly smoking, and then disruption of the alveoli or hypertrophy of the bronchial mucous gland occurs, to cause shortness of breath or increase cough or expectoration. A disease which has been once referred to as pulmonary emphysema (PE) and another disease which has been once referred to as chronic bronchitis (CB) are often complicated in varying proportions. The two diseases and obstructive pulmonary disease caused by the two diseases are referred to as chronic obstructive pulmonary disease (hereinafter referred to as COPD).

According to the estimation of the World Health Organization (WHO), three million people died worldwide from COPD in 2005, and COPD is the fourth most common cause of death. WHO predicts that the death from COPD increases by 30% in the next 10 years. According to the statistics of the Ministry of Health, Labor, and Welfare in Japan, the death from COPD is 1.3% of the total number of death among Japanese in 2005, and is the tenth most common cause of death and is the seventh most common cause of death only among males.

The most common cause of COPD is smoking. 90% of patients with COPD smoke (Non-patent Literature 1). The risk of COPD for smokers is higher more than six times than that for nonsmokers. About 10 to 15% of smokers develop COPD. About 50% of smokers in the elderly develop COPD. Examples of another cause include indoor air contamination, atmospheric contamination, inhalation of chemical substances or dust, heredity, childhood pneumonia and bronchitis, and the like.

A pathological condition of COPD is airflow limitation, that is, COPD is a disease, in which it is difficult to breathe. The nature of the pathological condition is caused by chronic inflammation of respiratory tract. Smoking and inhaled substances cause inflammation in the lung from the central airway to the peripheral airway at various levels. As a result, it is believed that protease-antiprotease imbalance, oxidant-antioxidant imbalance, or the like, causes disruption of the alveoli or hypertrophy of the bronchial mucous gland.

The COPD is a disease which is not healed since disruption of the respiratory tract irreversibly occurs. Abstaining from smoking, a drug therapy such as administration of a bronchodilator, an expectorant, or an antitussive, an oxygen therapy, or the like, only alleviates symptoms. The COPD is the most troubling disease.

Under such viewpoints, various ameliorating agents for COPD or ameliorating methods have been proposed (see Patent Literatures 11 and 12). Now, it is expected to make a more excellent ameliorating agent for COPD.

### PRIOR ART LIST

### PATENT LITERATURE

Patent Literature 1: Japanese Patent Application Laid-Open No. Hei 9-117279
Patent Literature 2: Japanese Patent Application Laid-Open No. Hei 9-52843
Patent Literature 3: Japanese Patent Application Laid-Open No. Hei 9-59178
Patent Literature 4: Japanese Patent Application Laid-Open No. Hei 9-110717
Patent Literature 5: Japanese Patent Application Laid-Open No. Hei 10-338645
Patent Literature 6: Japanese Patent Application Laid-Open No. 2001-2585
Patent Literature 7: Japanese Patent Application Laid-Open No. 2001-151695
Patent Literature 8: Japanese Patent Application Laid-Open No. 2006-169128
Patent Literature 9: Japanese Patent Application Laid-Open No. 2001-64199
Patent Literature 10: Japanese Patent Application Laid-Open No. 2007-099201
Patent Literature 11: Japanese Patent Application Laid-Open No. 2006-56890
Patent Literature 12: Japanese Patent Application Laid-Open No. 2008-189667

### NON-PATENT LITERATURE

Non-Patent Literature 1: Annual Review of Medicine, 40:1989, pp411-429

Inflammation in the lung tissue is considered to affect onset of COPD. Active oxygen such as superoxide anions, and iron complexes are involved in induction of the inflammation. Therefore, it is believed that elimination of active oxygen by SODs can suppress the induction and as a result, symptoms of COPD can be ameliorated.

From this viewpoints, the inventors have studied application of the previously proposed lecithinized superoxide dismutase (PC-SOD) having high affinity for cells to COPD. As a result, they have confirmed that the PC-SOD is very effective for amelioration in the symptoms of COPD, whereby the present invention has been completed.

### DISCLOSURE OF INVENTION

### PROBLEM TO BE SOLVED BY THE INVENTION

In view of the problems, it is an object of the present invention to provide a safe and effective ameliorating agent for use in treating COPD containing a PC-SOD as an active ingredient.

### MEANS FOR SOLVING THE PROBLEM

To solve the above-mentioned problems, one basic aspect of the present invention is an ameliorating agent for use in treating COPD containing as an active ingredient a lecithinized superoxide dismutase represented by the following general formula (I):

SOD'(Q-B)ₘ (I)

(in the formula SOD' represents a residue of the superoxide dismutase; Q represents a chemical crosslinking; B represents a residue without a hydrogen atom of a hydroxyl group of lysolecithin having the hydroxyl group at the 2-position of glycerol; m is the average number of bonds of lysolecithin to one molecule of superoxide dismutase and represents an integer of 1 or more).

The present invention is preferably an ameliorating agent for use in treating COPD, wherein the Q in the lecithinized superoxide dismutase represented by the formula (I) used in the present invention is -C(O)-(CH₂)ₙ-C(O)- (in the formula n represents an integer of 2 or more).

Further, the present invention is specifically an ameliorating agent for use in treating COPD, wherein SOD' is a residue of a human superoxide dismutase, and more specifically the SOD' is a residue of a modified superoxide dismutase in which an amino acid at a 111-position of amino acid sequence of the human superoxide dismutase is converted into S-(2-hydroxyethylthio)cysteine.

Most specifically, the present invention is an ameliorating agent for use in treating COPD, wherein the superoxide dismutase is a superoxide dismutase containing copper and zinc at an active center.

The present invention is an ameliorating agent for use in treating COPD, specifically containing a lecithinized superoxide dismutase and a stabilizing agent thereof, wherein the stabilizing agent is a sugar component, and particularly sucrose.

More specifically, the present invention is an ameliorating agent for use in treating COPD which has a form of injection or of inhalant.

### EFFECT OF THE INVENTION

In the present invention, since chronic inflammation occurs in the airway from the central bronchus to the peripheral trajectory as onset of COPD, and active oxygen such as superoxide anions, and iron complexes are involved in induction of the inflammation, elimination of active oxygen by SODs can effectively suppress the induction. As a result, effective amelioration therapy for COPD can be performed.

Under a circumstance where there have not been effective ameliorating agents for use in treating COPD heretofore, administration of specific PC-SOD is allowed to ameliorate the symptom. In this regard, medical effects are very unique.

The PC-SOD used in the present invention has higher affinity for cell membranes than the conventional SODs and higher ability for eliminating superoxide anions in the lesion. In addition, when a sugar component, and particularly sucrose, is contained together as the stabilizing agent, the PC-SOD itself becomes excellent in stability. Thus, the effect of a SOD having a short half-life is persistently exerted. In terms of ameliorating COPD, the PC-SOD is particularly excellent.

### BRIEF DESCRIPTION OF DRAWINGS

Fig. 1 is a view showing the whole number of cells in an alveolar lavage fluid in Test Example 1.
Fig. 2 is a view showing the results of alveolar macrophages as the number of inflammatory cells in Test Example 1.
Fig. 3 is a view showing the results of neutrophils as the number of inflammatory cells in Test Example 1.
Fig. 4 is a view showing the results of lymphocytes as the number of inflammatory cells in Test Example 1.
Fig. 5 is a micrograph of H&E stained lung tissues in Test Example 1.
Fig. 6 is a view showing the results of mean linear intercept (µm) in Test Example 1.
Fig. 7 is a view showing the whole number of cells in an alveolar lavage fluid in Test Example 2.
Fig. 8 is a view showing the results of alveolar macrophages as the number of inflammatory cells in Test Example 2.
Fig. 9 is a view showing the results of neutrophils as the number of inflammatory cells in Test Example 2.
Fig. 10 is a view showing the results of lymphocytes as the number of inflammatory cells in Test Example 2.
Fig. 11 is a micrograph of H&E stained lung tissues in Test Example 2.
Fig. 12 is a view showing the results of mean linear intercept (µm) in Test Example 2.

### BEST MODE(S) FOR CARRYING OUT THE INVENTION

In the lecithinized superoxide dismutase (PC-SOD) used for the ameliorating agent for use in treating COPD provided by the present invention, the term "lecithin" represents normal lecithin which means phosphatidylcholine, and the term "lysolecithin" represents a compound in which one molecule of fatty acid bound at the 2-position of glycerol in lecithin is removed and a hydroxyl group is bound to the carbon atom at the 2-position.

The PC-SOD used in the present invention can be usually obtained by binding one or more lecithin derivatives, in which a chemical crosslinking agent is bound to the hydroxyl group at the 2-position of lysolecithin, to the SOD. The PC-SOD can be represented by the following formula (I):

SOD'(Q-B)m (I)

(in the formula SOD' represents a residue of the superoxide dismutase; Q represents a chemical crosslinking; B represents a residue without a hydrogen atom of a hydroxyl group of lysolecithin having the hydroxyl group at the 2-position of glycerol; m is the average number of bonds of lysolecithin to one molecule of superoxide dismutase and represents an integer of 1 or more).

The SOD' used herein is not particularly limited to an origin thereof as long as an essential function of decomposing active oxygen (O₂⁻) in the living organism is exerted. SOD residues derived from various plants, animals, or microorganisms can be widely used. However, in view of application for medicines, it is preferable that antigenicity in the living organism be reduced as much as possible. Accordingly, as the SOD' used, it is preferable to suitably select appropriate SOD residues depending on subjects, to which the ameliorating agent for use in treating COPD of the present invention is administrated.

For example, the SOD' is one attempting to be administrated to actual patients with COPD as the subject. Therefore, in order to reduce antigenicity in the living organism due to the administration as much as possible, human-derived SOD residues are preferably used. Accordingly, as the ameliorating agent for use in treating COPD of the present invention, in view of antigenicity, the human-derived SOD may be used better.

As the human-derived SOD, a human-derived Cu/Zn-SOD (human-derived SOD containing copper and zinc at the active center; hereinafter may be abbreviated as human Cu/Zn-SOD) is particularly preferably used. This is because the human Cu/Zn-SOD is expressed in a large amount in cells, the production technology therefor based on a genetic engineering method has been already established, whereby the human Cu/Zn-SOD can be prepared in a large amount.

The human Cu/Zn-SOD includes: a natural human Cu/Zn-SOD produced from human tissues or cultured cells; a human Cu/Zn-SOD produced by the gene engineering method; a recombinant human Cu/Zn-SOD having substantially the same amino acid sequence as in the natural human Cu/Zn-SOD; a SOD where partial amino acids in amino acid sequences of these human Cu/Zn-SODs are deleted, added, substituted, or chemically modified or changed; and the like. Any human Cu/Zn-SOD may be used.

Among them, a human Cu/Zn-SOD in which an amino acid (cysteine: Cys) at the 111-position of amino acid sequence of natural human Cu/Zn-SOD has been converted into S-(2-hydroxyethylthio)cysteine is preferable. Such a human Cu/Zn-SOD is described in detail in Japanese Patent Application Laid-Open No. Hei 9-117279, and can be obtained by the method described therein.

Accordingly, preparation of human Cu/Zn-SOD described in Japanese Patent Application Laid-Open No. Hei 9-117279 is partially incorporated herein. In the case of the PC-SOD used in the present invention, these human Cu/Zn-SODs can be obtained as a material.

In the PC-SOD represented by the formula (I) used in the present invention, "a residue without a hydrogen atom of a hydroxyl group of lysolecithin having the hydroxyl group at the 2-position of glycerol" shown as B is specifically represented by the following formula (II):

-O-CH(CH₂OR)[CH₂OP(O)(O⁻)(OCH₂CH₂N⁺(CH₃)₃)] (II)

(in the formula R is a fatty acid residue (acyl group)).

The fatty acid residue (acyl group) represented by R is preferably a saturated or unsaturated C10-C28 fatty acid residue, more preferably a myristoyl group, a palmitoyl group, a stearoyl group, an icosanoyl group, a docosanoyl group, and another saturated C14-C22 fatty acid residue, and particularly preferably a palmitoyl group which is a saturated C16 fatty acid residue.

The chemical crosslinking represented by Q in the general formula (I) is not particularly limited as long as a SOD and lecithin can be crosslinked to be chemically (covalently) bound with each other. Such a chemical crosslinking is particularly preferably a residue: -C(O)-(CH₂)ₙ-C(O)- (in the formula n represents an integer of 2 or more). This residue is a residue without hydroxyl groups at both the ends of a linear dicarboxylic acid represented by formula: HO-C(O)-(CH₂)ₙ-C(O)-OH, an anhydride, ester, or halide thereof, or the like (provided that in the case of the anhydride, ester, and halide, a moiety corresponding to the hydroxyl groups at both the ends).

When Q in the general formula (I) is the above-described linear dicarboxylic acid residue, one end of Q is bound to oxygen of the hydroxyl group of lysolecithin residue represented by the formula (II) through an ester bond. Further, the other end of Q whose one end has formed the ester bond is directly bound to an amino group of SOD through an amide bond, or the like.

In the residue of the above-described chemical crosslinking, n is an integer of 2 or more, and preferably an integer of 2 to 10.

In the formula (I), m represents the average number of bond of lysolecithin to one molecule of SOD. Accordingly, m is an integer of 1 or more, preferably 1 to 12, and particularly preferably 4.

A method for producing PC-SOD used in the present invention, that is, a method for binding a lecithin derivative with a SOD, and preferably with a human Cu/Zn-SOD can be performed, for example, by using the method described in Japanese Patent Application Laid-Open No. Hei 9-117279.

A schematically shown chemical structure of the preferable PC-SOD is particularly preferably the following PC-SOD. 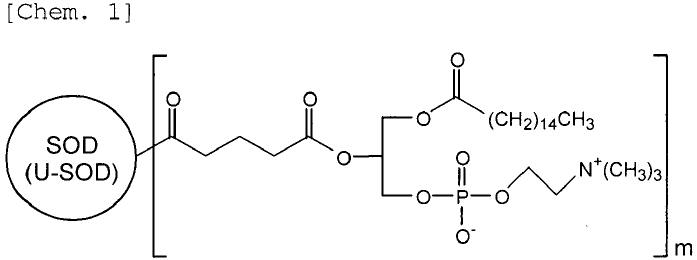 (wherein m is the number of bound lecithin derivatives).

In other words, the PC-SOD is obtained by covalently binding an average of four molecules of lecithin derivative to a free amino group of human Cu/Zn-SOD produced by genetic recombination using E. coli as a host cell.

It is preferable that the PC-SOD used in the ameliorating agent for use in treating COPD of the present invention be purified to such an extent that it is usable as a medicine and do not substantially contain substances which are not permitted to be mixed as a medicine. For example, it is preferable that as the PC-SOD, a purified PC-SOD having a specific SOD activity of 2,500 U/mg or more, and more preferably a purified PC-SOD having a specific SOD activity of 3,000 U/mg or more can be used.

In the present invention, 1 U (unit) represents an enzyme amount of PC-SOD which inhibits 50% of NBT (nitro blue tetrazolium) reduction rate as measured using NBT under a condition of pH 7.8 and 30°C in accordance with a method described in J. Biol. Chem., vol. 244, No. 22 6049-6055 (1969).

The ameliorating agent for use in treating COPD of the present invention is an ameliorating agent for use in treating COPD containing the PC-SOD thus prepared as an active ingredient, and preferably an ameliorating agent for use in treating COPD containing the PC-SOD and a stabilizing agent. Examples of the stabilizing agent include a sugar component. The sugar component is not particularly limited as long as it can be used pharmaceutically, and sucrose is preferable. Therefore, the most preferable ameliorating agent for use in treating COPD provided by the present invention is a composition containing a PC-SOD and sucrose. As sucrose, sucrose purified to such an extent that it is used as a medicine is preferably used, and sucrose processed by activated carbon is particularly preferably used. The ameliorating agent for use in treating COPD can be prepared as a composition, in which use of such sucrose with a PC-SOD can prevent reduction in the activity of the PC-SOD due to long term storage, the stability is high, and the property is particularly favorable even if it is lyophilized.

A mixing ratio of the PC-SOD to sucrose in the ameliorating agent for use in treating COPD of the present invention can be suitably determined depending on an administration amount, a form of the formulation, or the like, and is not particularly limited. However, a weight ratio of the PC-SOD to sucrose is preferably within a range of about 0.1/100 to 80/100, and more preferably about 0.4/100 to 60/100.

To the ameliorating agent for use in treating COPD of the present invention, another medical active component and a commonly-used formulation component such as an excipient, a binder, a lubricant, a colorant, a disintegrator, a buffer, a tonicity adjusting agent, a preservative, and a soothing agent can be added as long as they do not affect the activity of PC-SOD and the effect of formulation.

The ameliorating agent for use in treating COPD provided by the present invention can be prepared using PC-SOD and sucrose by a commonly-used method which is pharmaceutically known. PC-SOD used for a formulation composition of the present invention preferably has a solution form, a freezing form, or a lyophilization form.

In one aspect, the ameliorating agent for use in treating COPD provided by the present invention can be preferably administrated in the form of injection. The injection preferably has a form of solution, suspension, emulsion, or solid formulation which is dissolved before use. These formulations can be prepared in accordance with a method described in General Rules for Preparations of The Japanese Pharmacopoeia.

In another aspect, the ameliorating agent for use in treating COPD provided by the present invention can be preferably administrated in the form of inhalant.

Such an inhalant means a pharmaceutical composition for delivery to the trachea, bronchus, lung, and the like, suitably a composition suitable for a nasal drop, or administration through the nose or lung, and particularly a composition suitable for administration through the lung.

The inhalant can be produced in the form of powder, solution, or suspension using the above-described PC-SOD as an active ingredient.

When an inhalant is produced in a powder form, the PC-SOD as an active ingredient is pulverized as it is or with additives such as excipients, lubricants, binders, disintegrators, stabilizing agents, and correctives, to produce the inhalant.

When the inhalant is produced as a solution or suspension, PC-SOD is dissolved or suspended in water or a mixture of water and an auxiliary solvent, for example, an alcohol auxiliary solvent such as ethanol, propylene glycol, and polyethylene glycol, to produce the inhalant. Such a solution or suspension can additionally contain antiseptics, solubilizers, buffers, tonicity adjusting agents, absorption promoters, thickeners, and the like.

The inhalant produced as described above is directly administrated inside the nasal or mouth cavity or to the trachea, bronchi, lung, or the like in a nebulized form by using common means in the field of inhalant, for example, a dropper, a pipette, a cannula, or a sprayer such as an atomizer or a nebulizer. In the case of using a sprayer, the inhalant can be administrated by spraying it as an aerosol in the form of pressure bag with an appropriate propellant (e.g., gases of chlorofluorocarbons such as dichlorofluoromethane, trichlorofluoromethane, or dichlorotetrafluoroethane, carbon dioxide, or the like), or by using a nebulizer.

The amount of PC-SOD which is an active ingredient in the ameliorating agent for use in treating COPD of the present invention and the administration amount of the formulation are varied depending on a method for preparing the formulation, a dosage form, a target disease degree, or age or body weight of a patient, but not particularly limited. For example, as a clinical amount, 0.5 to 100 mg (1,500 to 300,000 U) daily per adult can be exemplified. Further, the number of doses is not particularly limited, but the administration can be performed once or more daily.

### EXAMPLES

Hereinafter, the present invention will be described in detail by description of specific Test Examples and Examples. However, the present invention is not limited to the description.

### Test Example 1: Effect of PC-SOD for porcine pancreas elastase-induced COPD model mouse (intravenous administration)

### [Processes]

To 6 to 8 weeks old BALB/c mice, 50 µg of porcine pancreas elastase was intratracheally administrated per mouse, to produce lung injury.

Each PC-SOD (1.5 and 3.0 kU/kg) was intravenously administrated once daily. After 3 days, an alveolar lavage fluid was collected, and the whole cells were counted.

Further, the cells were stained by Diff-Quik stain, and inflammatory cells were counted.

Then, the mice were sacrificed, and slices of a lung tissue were produced. The slices were stained by H&E stain and a stained image (x 40) was taken with an electron microscope.

A mean linear intercept (µm) was measured from the H&E stained image.

### [Results]

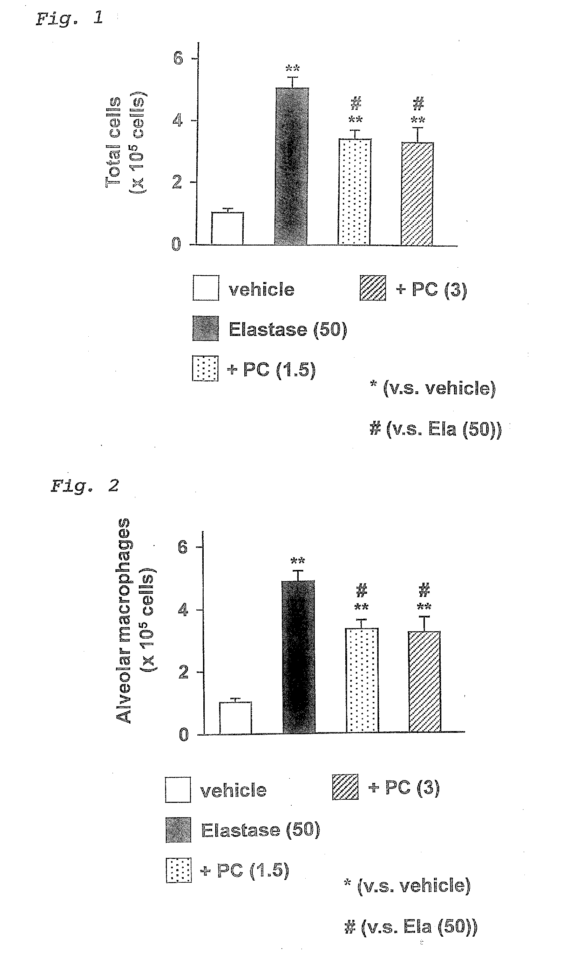

1. Fig. 1 shows the whole number of cells in the alveolar lavage fluid.
   As seen from the results shown in the drawings, administration of porcine pancreas elastase increases the whole number of cells. Therefore, increase in inflammatory cells is predicted. Further, since PC-SOD decreases this increase, exertion of antiinflammation effect by PC-SOD is predicted.
2. Figs. 2 to 4 show the number of each inflammatory cell.

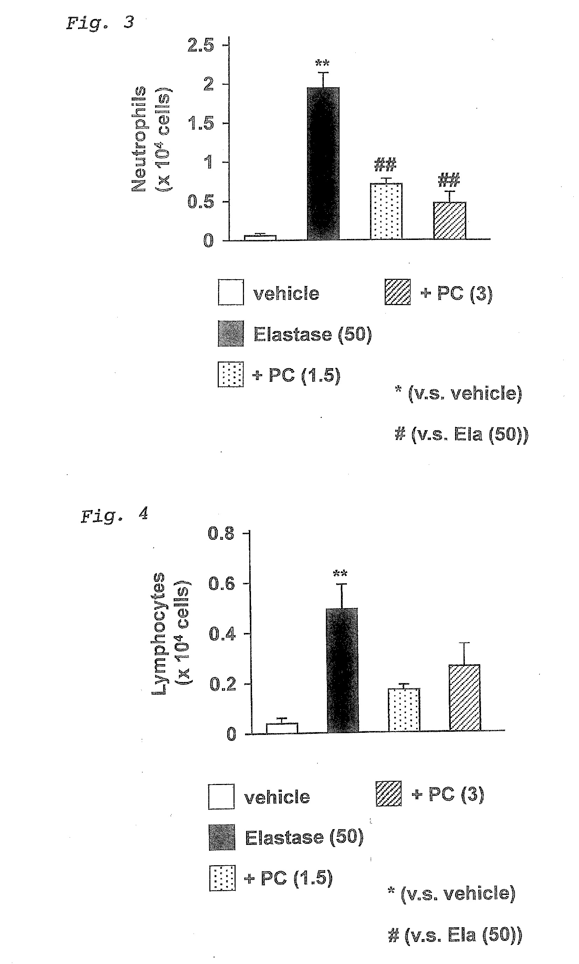

   Fig. 2 shows the results of alveolar macrophages, Fig. 3 shows the results of neutrophils, and Fig. 4 shows the results of lymphocytes.
   As seen from the results shown in the drawings, intravenous administration of 1.5 and 3.0 kU/kg of PC-SOD shows decrease in inflammatory cells. It is clear that the PC-SOD shows significantly antiinflammation effects.

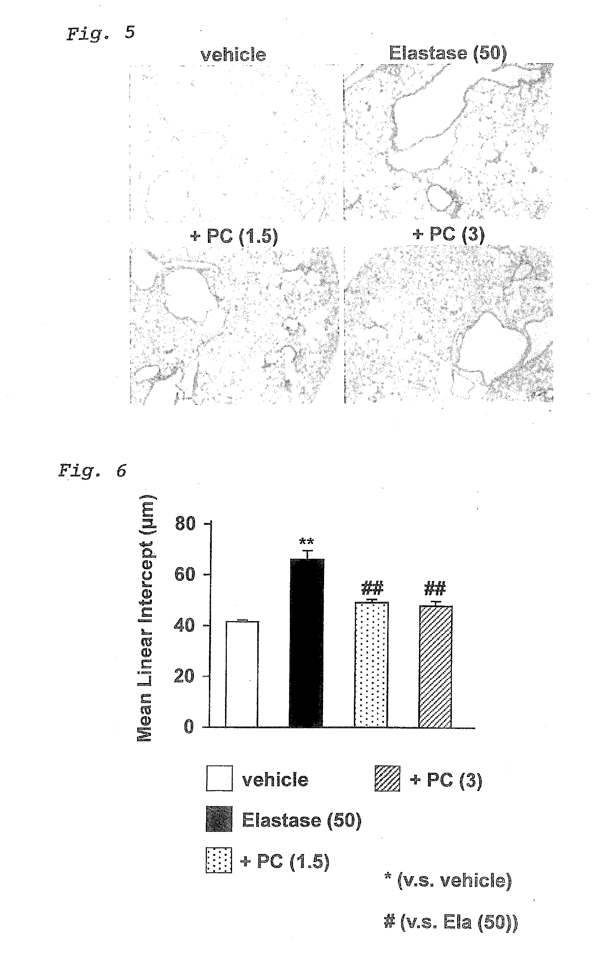

3. Fig. 5 shows the results of micrograph of H&E stained lung tissues, and Fig. 6 shows the results of mean linear intercept (µm).

As seen from the results shown in the drawings, intravenous administration of 1.5 and 3.0 kU/kg of PC-SOD suppresses extension of mean linear intercept depending on elastase. It is clear that the PC-SOD suppresses lung injury.

### Test Example 2: Effect of PC-SOD for porcine pancreas elastase-induced COPD model mouse (inhalation administration)

### [Processes]

To 6 to 8 weeks old BALB/c mice, 50 µg of porcine pancreas elastase was intratracheally administrated per mouse, to produce lung injury.

Each inhalant containing PC-SOD (30 and 60 kU/Chamber) with each concentration was administrated by inhalation once daily. After 3 days, an alveolar lavage fluid was collected, and the whole cells were counted.

Further, the cells were stained by Diff-Quik stain, and inflammatory cells were counted.

Then, the mice were sacrificed, and slices of a lung tissue were produced. The slices were stained by H&E stain and a stained image (x 40) was taken with an electron microscope.

A mean linear intercept (µm) was measured from the H&E stained image.

### [Results]

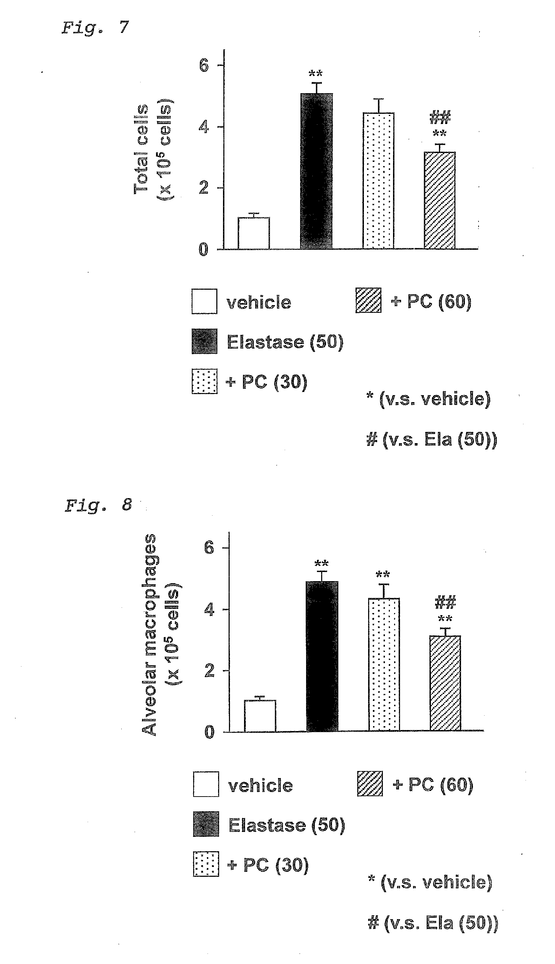

1. Fig. 7 shows the whole number of cells in the alveolar lavage fluid.
   As seen from the results shown in the drawings, PC-SOD decreases the whole number of cells which is increased depending on administration of porcine pancreas elastase. Therefore, exertion of antiinflammation effect by PC-SOD is predicted.
2. Figs. 8 to 10 show the number of each inflammatory cell.

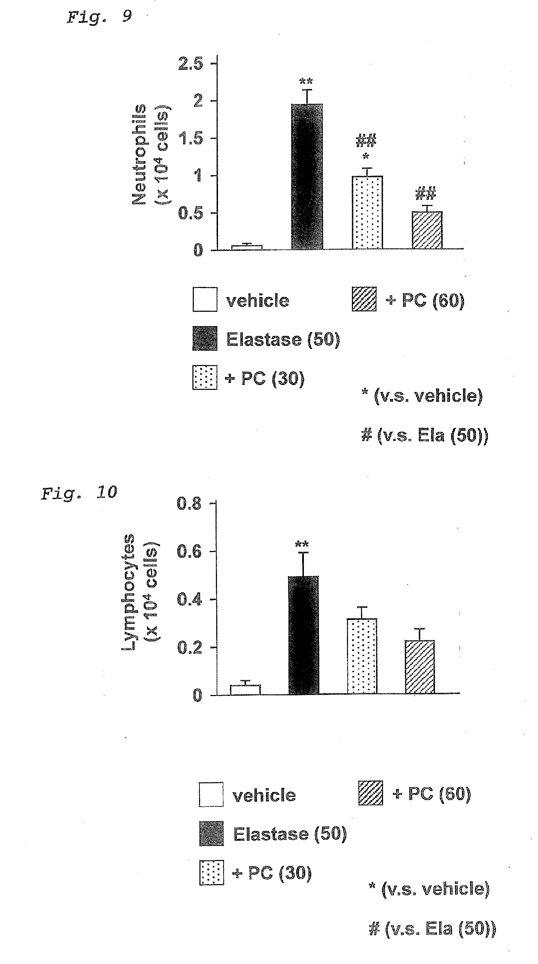

   Fig. 8 shows the results of alveolar macrophages, Fig. 9 shows the results of neutrophils, and Fig. 10 shows the results of lymphocytes.
   As seen from the results shown in the drawings, it is clear that inhalation administration (through the lung) of inhalant containing 30 and 50 kU/Chamber of PC-SOD shows significantly antiinflammation effects.

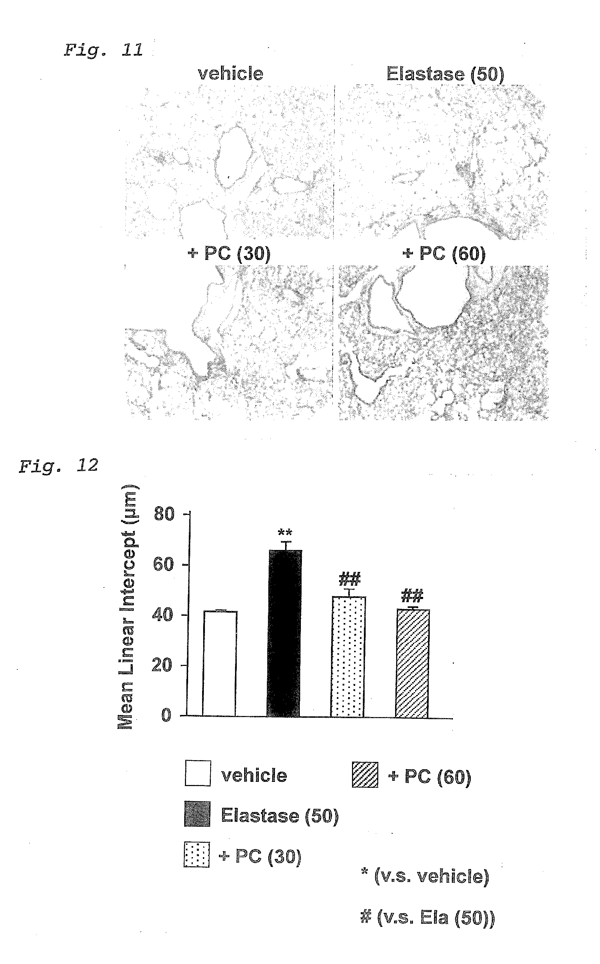

3. Fig. 11 shows the results of micrograph of H&E stained lung tissues, and Fig. 12 shows the results of mean linear intercept (µm).

As seen from the results shown in the drawings, inhalation administration (through the lung) of inhalant containing 30 and 50 kU/Chamber of PC-SOD suppresses extension of mean linear intercept depending on elastase. It is clear that the PC-SOD suppresses lung injury.

### Formulation Example 1: intravenous injection

1%(w/w) of PC-SOD, 10%(w/w) of sucrose, and 0.05%(w/w) of benzalkonium chloride were dissolved in an aqueous solution of 5% xylitol, and the solution was lyophilized. To the obtained lyophilized product, 0.5% carmellose which was separately filled in a vial or water for injection was added, to obtain an intravenous injection.

### Example 2: Inhalant

### Liquid formulation for inhalation (1)

1%(w/w) of PC-SOD, 10%(w/w) of sucrose, and 0.05%(w/w) of benzalkonium chloride were dissolved in an aqueous solution of 5% xylitol, to prepare a liquid formulation for inhalation.

### Liquid formulation for inhalation (2)

1%(w/w) of PC-SOD, 10%(w/w) of sucrose, 0.05%(w/w) of benzalkonium chloride, 10%(w/w) of polyethylene glycol, 20%(w/w) of propylene glycol, and the balance of purified water were used to prepare a liquid formulation for inhalation.

### Powder formulation for inhalation

5%(w/w) of PC-SOD and the balance of sucrose (fine powder) were used to prepare a powder formulation for inhalation.

### INDUSTRIAL APPLICABILITY

As described above, the ameliorating agent for use in treating COPD provided by the present invention contains a specific PC-SOD as an active ingredient, and has higher affinity for cell membranes than the conventional SOD and higher ability to eliminate superoxide anions in the lesion. Further, when the PC-SOD and sucrose are contained together, the PC-SOD itself becomes excellent in stability. Thus, the effect of a SOD having a short half-life is continuously exerted. The effect of SOD eliminates active oxygen such as superoxide anions which induce cell dysfunction to effectively suppress the induction. As a result, amelioration useful for COPD can be performed, and has a great value in medical care.

## Claims

1. An ameliorating agent for use in treating chronic obstructive pulmonary disease, containing as an active ingredient a lecithinized superoxide dismutase represented by the following general formula (I):
SOD'(Q-B)ₘ (I)
(in the formula SOD' represents a residue of the superoxide dismutase; Q represents a chemical crosslinking; B represents a residue without a hydrogen atom of a hydroxyl group of lysolecithin having the hydroxyl group at the 2-position of glycerol; m is the average number of bonds of lysolecithin to one molecule of superoxide dismutase and represents an integer of 1 or more).

2. The ameliorating agent for use in treating chronic obstructive pulmonary disease according to claim 1, wherein the Q in the formula (I) is -C(O)-(CH₂)ₙ-C(O)- (in the formula n represents an integer of 2 or more).

3. The ameliorating agent for use in treating chronic obstructive pulmonary disease according to claim 1 or 2, wherein the SOD' is a residue of a human superoxide dismutase.

4. The ameliorating agent for use in treating chronic obstructive pulmonary disease according to claim 1 or 2, wherein the SOD' is a residue of a modified superoxide dismutase in which an amino acid at a 111-position of amino acid sequence of the human superoxide dismutase is converted into S-(2-hydroxyethylthio)cysteine.

5. The ameliorating agent for use in treating chronic obstructive pulmonary disease according to claim 3 or 4, wherein the superoxide dismutase is a superoxide dismutase containing copper and zinc at an active center.

6. The ameliorating agent for use in treating chronic obstructive pulmonary disease according to any one of claims 2 to 5, wherein n is an integer of 2 to 10.

7. The ameliorating agent for use in treating chronic obstructive pulmonary disease according to any one of claims 1 to 6, wherein m is an integer of 1 to 12.

8. The ameliorating agent for use in treating chronic obstructive pulmonary disease according to any one of claims 1 to 7, further containing a stabilizing agent.

9. The ameliorating agent for use in treating chronic obstructive pulmonary disease according to claim 8, wherein the stabilizing agent is a sugar.

10. The ameliorating agent for use in treating chronic obstructive pulmonary disease according to claim 9, wherein the sugar is sucrose.

11. The ameliorating agent for use in treating chronic obstructive pulmonary disease according to claim 10, wherein the sucrose is a sucrose having been subjected to activated carbon process.

12. The ameliorating agent for use in treating chronic obstructive pulmonary disease according to claim 1, having a form of injection.

13. The ameliorating agent for use in treating chronic obstructive pulmonary disease according to claim 1, having a form of inhalant.

## Patentansprüche

1. Linderungsmittel zur Verwendung in der Behandlung chronisch-obstruktiver Lungenerkrankung, enthaltend als aktives Ingrediens eine lecithinisierte Superoxid-Dismutase, dargestellt durch die folgende allgemeine Formel (I):
SOD'(Q-B)ₘ (I)
(in der Formel stellt SOD' einen Rest der Superoxid-Dismutase dar, stellt Q eine chemische Quervernetzung dar, stellt B einen Rest ohne ein Wasserstoffatom einer Hydroxylgruppe von Lysolecithin mit der Hydroxylgruppe an der 2-Position von Glycerin dar, ist m die durchschnittliche Anzahl von Bindungen von Lysolecithin an ein Molekül der Superoxid-Dismutase dar, und stellt eine ganze Zahl von 1 oder größer dar).

2. Linderungsmittel zur Verwendung bei der Behandlung chronisch-obstruktiver Lungenerkrankung gemäß Anspruch 1, wobei das Q in der Formel (I) -C(O)-(CH₂)ₙ-C(O)- ist (in der Formel stellt n eine ganze Zahl von 2 oder größer dar).

3. Linderungsmittel zur Verwendung bei der Behandlung chronisch-obstruktiver Lungenerkrankung gemäß Anspruch 1 oder 2, wobei das SOD' ein Rest einer humanen Superoxid-Dismutase ist.

4. Linderungsmittel zur Verwendung bei der Behandlung chronisch-obstruktiver Lungenerkrankung gemäß Anspruch 1 oder 2, wobei das SOD' ein Rest einer modifizierten Superoxid-Dismutase ist, bei der eine Aminosäure an einer 111-Position der Aminosäuresequenz der humanen Superoxid-Dismutase zu S-(2-Hydroxyethylthio)cystein umgewandelt ist.

5. Linderungsmittel zur Verwendung bei der Behandlung chronisch-obstruktiver Lungenerkrankung gemäß Anspruch 3 oder 4, wobei die Superoxid-Dismutase eine Superoxid-Dismutase ist, die an einem aktiven Zentrum Kupfer und Zink enthält.

6. Linderungsmittel zur Verwendung bei der Behandlung chronisch-obstruktiver Lungenerkrankung gemäß einem der Ansprüche 2 bis 5, wobei n eine ganze Zahl von 2 bis 10 ist.

7. Linderungsmittel zur Verwendung bei der Behandlung chronisch-obstruktiver Lungenerkrankung gemäß einem der Ansprüche 1 bis 6, wobei m eine ganze Zahl von 1 bis 12 ist.

8. Linderungsmittel zur Verwendung bei der Behandlung chronisch-obstruktiver Lungenerkrankung gemäß einem der Ansprüche 1 bis 7, welches außerdem ein stabilisierendes Agens enthält.

9. Linderungsmittel zur Verwendung bei der Behandlung chronisch-obstruktiver Lungenerkrankung gemäß Anspruch 8, wobei das stabilisierende Agens ein Zucker ist.

10. Linderungsmittel zur Verwendung bei der Behandlung chronisch-obstruktiver Lungenerkrankung gemäß Anspruch 9, wobei der Zucker Saccharose ist.

11. Linderungsmittel zur Verwendung bei der Behandlung chronisch-obstruktiver Lungenerkrankung gemäß Anspruch 10, wobei die Saccharose eine Saccharose ist, welche einem Aktivkohleprozess unterzogen worden ist.

12. Linderungsmittel zur Verwendung bei der Behandlung chronisch-obstruktiver Lungenerkrankung gemäß Anspruch 1, welches sich in der Form einer Injektion befindet.

13. Linderungsmittel zur Verwendung bei der Behandlung chronisch-obstruktiver Lungenerkrankung gemäß Anspruch 1, welches sich in der Form eines Inhalans befindet.

## Revendications

1. Agent d'amélioration pour utilisation dans le traitement de la bronchopneumopathie chronique obstructive, contenant en tant que substance active une superoxyde dismutase lécithinisée représentée par la formule générale (I) suivante :
SOD'(Q-B)ₘ (I)
(dans la formule SOD' représente un résidu de la superoxyde dismutase ; Q représente une réticulation chimique ; B représente un résidu sans atome d'hydrogène d'un groupe hydroxyle de lysolécithine ayant le groupe hydroxyle en position 2 du glycérol ; m est le nombre moyen de liaisons de lysolécithine pour une molécule de superoxyde dismutase et représente un nombre entier de 1 ou plus).

2. Agent d'amélioration pour utilisation dans le traitement de la bronchopneumopathie chronique obstructive selon la revendication 1, dans lequel le Q dans la formule (I) est un groupe -C(O)-(CH₂)ₙ-C(O)- (dans la formule n représente un nombre entier de 2 ou plus).

3. Agent d'amélioration pour utilisation dans le traitement de la bronchopneumopathie chronique obstructive selon la revendication 1 ou 2, dans lequel le SOD' est un résidu d'une superoxyde dismutase humaine.

4. Agent d'amélioration pour utilisation dans le traitement de la bronchopneumopathie chronique obstructive selon la revendication 1 ou 2, dans lequel le SOD' est un résidu d'une superoxyde dismutase modifiée dans laquelle un acide aminé en position 111 de la séquence d'acides aminés de la superoxyde dismutase humaine est converti en S-(2-hydroxyéthylthio)cystéine.

5. Agent d'amélioration pour utilisation dans le traitement de la bronchopneumopathie chronique obstructive selon la revendication 3 ou 4, dans lequel la superoxyde dismutase est une superoxyde dismutase contenant du cuivre et du zinc en tant que centre actif.

6. Agent d'amélioration pour utilisation dans le traitement de la bronchopneumopathie chronique obstructive selon l'une quelconque des revendications 2 à 5, dans lequel n est un nombre entier de 2 à 10.

7. Agent d'amélioration pour utilisation dans le traitement de la bronchopneumopathie chronique obstructive selon l'une quelconque des revendications 1 à 6, dans lequel m est un nombre entier de 1 à 12.

8. Agent d'amélioration pour utilisation dans le traitement de la bronchopneumopathie chronique obstructive selon l'une quelconque des revendications 1 à 7, contenant en outre un agent stabilisant.

9. Agent d'amélioration pour utilisation dans le traitement de la bronchopneumopathie chronique obstructive selon la revendication 8, dans lequel l'agent stabilisant est un sucre.

10. Agent d'amélioration pour utilisation dans le traitement de la bronchopneumopathie chronique obstructive selon la revendication 9, dans lequel le sucre est un sucrose.

11. Agent d'amélioration pour utilisation dans le traitement de la bronchopneumopathie chronique obstructive selon la revendication 10, dans lequel le sucrose est un sucrose ayant été soumis à un procédé au charbon actif.

12. Agent d'amélioration pour utilisation dans le traitement de la bronchopneumopathie chronique obstructive selon la revendication 1, se présentant sous la forme d'un produit pour injection.

13. Agent d'amélioration pour utilisation dans le traitement de la bronchopneumopathie chronique obstructive selon la revendication 1, se présentant sous la forme d'un produit pour inhalation.
